(19) Europäisches Patentamt
European Patent Office
Office européen des brevets

(11) Publication number: **0 173 664**
**A2**

(12) # EUROPEAN PATENT APPLICATION

(21) Application number: **85850258.6**

(22) Date of filing: **09.08.85**

(51) Int. Cl.⁴: **C 07 D 213/26**, C 07 D 213/28, C 07 D 401/12

(30) Priority: **10.08.84 SE 8404065**

(43) Date of publication of application: **05.03.86 Bulletin 86/10**

(84) Designated Contracting States: **AT BE CH DE FR GB IT LI LU NL SE**

(71) Applicant: **Aktiebolaget Hässle, Kärragatan 5, S-431 83 Mölndal (SE)**

(72) Inventor: **Bränström, Arne Elof, Anders Mattssonsgatan 13B, S-415 06 Göteborg (SE)**
Inventor: **Carlsson, Stig Ake Ingemar, Vallmovägen 3, S-435 00 Mölnlycke (SE)**
Inventor: **Källsson, Britt Inger Monica, Dalbogatan 3, S-431 38 Mölndal (SE)**
Inventor: **Lindberg, Per Lennart, Knapehall 64, S-436 00 Askim (SE)**

(74) Representative: **Danielsson, Sten Ove et al, AB ASTRA Patent and Trademark Department, S-151 85 Södertälje (SE)**

(54) **Biologically active benzimidazole compounds and process for their preparation.**

(57) Novel compounds of the formula

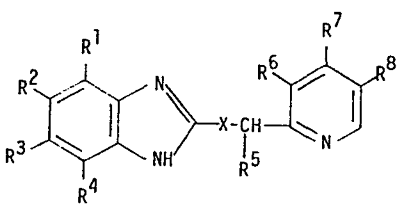

pharmaceutical compositions containing such compounds as active ingredient, and the use of the compounds in medicine.

EP 0 173 664 A2

0173664

Novel Biologically Active Compounds

TITLE MODIFIED
see front page

DESCRIPTION

Field of the invention

The object of the present invention is to provide novel compounds, and therapeutically acceptable salts thereof, which inhibit exogenously or endogenously stimulated gastric acid secretion and provide gastrointestinal cytoprotective effects and thus can be used in the prevention and treatment of peptic ulcer.

The present invention also relates to the use of the compounds of the invention or therapeutically acceptable salts thereof, for inhibiting gastric acid secretion as well as for providing gastrointestinal cytoprotective effects in mammals and man. In a more general sense, the compounds of the invention may be used for prevention and treatment of gastrointestinal inflammatory diseases in mammals and man, including e.g. gastritis, gastric ulcer, and duodenal ulcer. Furthermore, the compounds may be used for prevention and treatment of other gastrointestinal disorders, where cytoprotective and/or gastric antisecretory effect is desirable e.g. in patients with gastrinomas, in patients with acute upper gastrointestinal bleeding, and in patients with a history of chronic and excessive ethanol consumption. The invention also relates to pharmaceutical compositions containing at least one compound of the invention, or a therapeutically acceptable salt thereof, as active ingredient. In a further aspect, the invention relates to processes for preparation of such new compounds and to novel intermediates in the preparation of the compounds of the invention.

Prior art

Benzimidazole derivatives intended for inhibiting gastric acid secretion are disclosed in the British patent specifications 1 500 043 and 1 525 958, in the US patent 4 182 766, in the European patent specification 0 005 129, in the Belgian patent specification 890 024, and in the European patent application with publication no. 0 080 602. Benzimidazole derivatives proposed for use in the treatment or prevention of special gastrointestinal inflammatory disease are disclosed in the European patent application with publication no. 0 045 200.

The invention

It has been found that the compounds of the formula

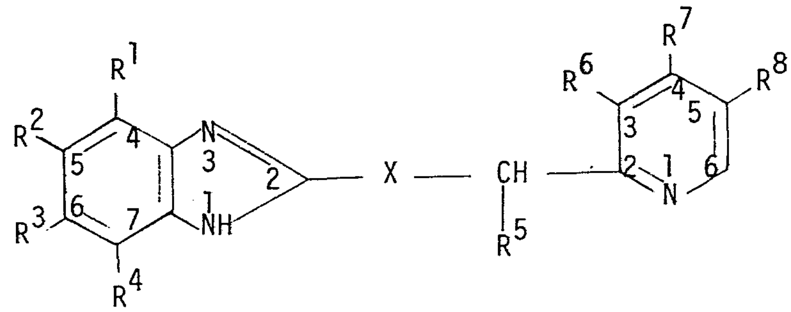

I

and therapeutically acceptable salts thereof, in which formula

X is -S- or $-\overset{\overset{O}{\uparrow}}{S}-$;

$R^1$, $R^2$, $R^3$ and $R^4$, which are the same or different, are

(a) H

(b) alkyl containing 1-6 carbon atoms

(c) cycloalkyl containing 3-7 carbon atoms

(d) alkoxy containing 1-6 carbon atoms

(e) alkoxyalkoxy containing 1-3 carbon atoms in each alkyl part

(f) halogen

(g) $CF_3$

(h) aryl

(i) aryloxy

or

(j) alkanoyl containing 2-7 carbon atoms;

$R^5$ is H, $CH_3$ or $C_2H_5$;

$R^6$ and $R^8$, which are the same or different, are

(a) H

(b) alkyl containing 1-6 carbon atoms;

and wherein

$R^7$ is $-(O)_m-(CH_2)_n-$ 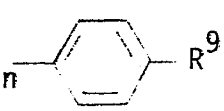

wherein

m is 0 or 1
n is 0, 1 or 2
$R^9$ is H or $CH_3$,

provided that when m, n, $R^9$, X, $R^1$, $R^2$, $R^3$, $R^4$, $R^5$, $R^6$ and $R^8$ are combined as follows: m is 1; n is 2; $R^9$ is H; X is SO; $R^1$, $R^3$ and $R^4$ are H; $R^6$ and $R^8$ are $CH_3$; and $R^5$ is H; then $R^2$ is other than $OCH_3$,

are effective as gastrointestinal cytoprotectives and as inhibitors of gastric acid secretion in mammals and man as stated above.

In particular the compounds of the formula I wherein at the same time m is 1 and n is 0 are remarkably stable compared to analogous compounds known in the prior art with other substituents in the two aromatic rings, for example analogues containing a pyridine ring substituted either by a 4-alkoxy group, or by 3,4-dimethyl or 4,5-dimethyl groups.

This high stability is particularly pronounced at pH 5-7, although the for the biological activity necessarily high acid lability (pH 1-3) is essentially retained. The high stability at pH 5-7 combined with the high reactivity at pH 1-3 which are characteristic of the said compounds with the formula I wherein m is 1 and n is 0, is very important for giving a desirable high biological selectivity of the said compounds of the invention and their salts.

Illustrative examples of the various radicals in the formula I are as follows. It will be understood that the expression "alkyl" includes straight and branched structures, that the expression "cycloalkyl" includes cycloalkyl-alkyl structures containing 4-7 carbon atoms, and that the expression "alkoxy" includes straight, branched and cyclic structures.

Halogen: F, Cl, Br, I

Alkyl: $CH_3$, $C_2H_5$, n-$C_3H_7$, i-$C_3H_7$, n-$C_4H_9$, sec.-$C_4H_9$, iso.-$C_4H_9$, tert.-$C_4H_9$, n-$C_5H_{11}$, n-$C_6H_{13}$,

Cycloalkyl: 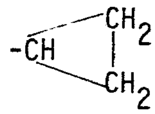 , 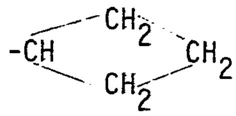 , 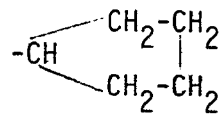 ,

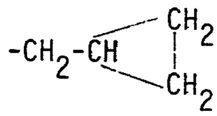 , 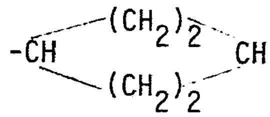

Alkoxy: $-OCH_3$ , $-OC_2H_5$ , $-O-n-C_3H_7$ , $-O-i-C_3H_7$ , $-O-n-C_4H_9$,
$-O-iso-C_4H_9$ , $-O-sec.-C_4H_9$ , $-O-tert.-C_4H_9$ , $-O-n-C_5H_{11}$ ,
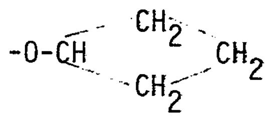 , 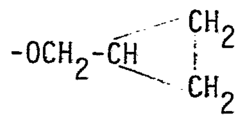 , 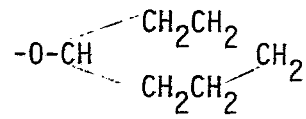

Alkoxyalkoxy: $-OCH_2OCH_3$ , $-OCH_2CH_2OCH_3$ , $-OCH_2CH_2OCH_2CH_3$ ,
$-OCH_2CH_2CH_2OCH_2CH_2CH_3$

Aryl: 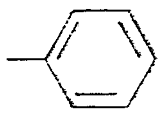

Aryloxy: 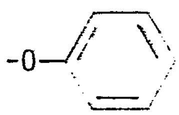

Alkanoyl: 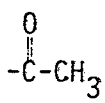 , 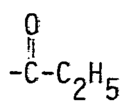 , 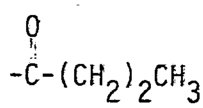 , 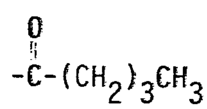 ,
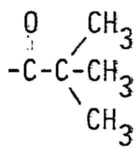 , 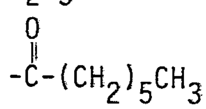

Illustrative examples of the radical $R^7$ are

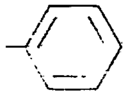 , 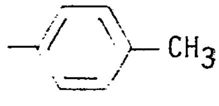 , 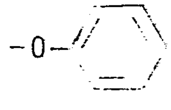 , 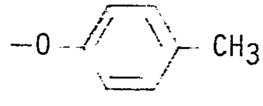 ,

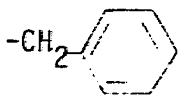 , 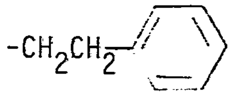 , 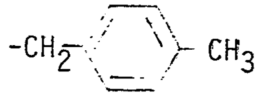 , 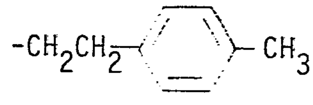 ,

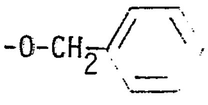 , 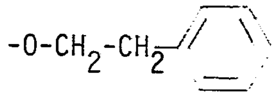 , 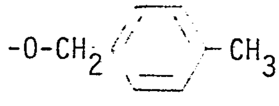 , 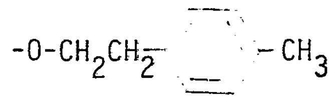 .

The compounds of the invention that are sulfoxides (X=SO) have an asymmetric centre in the sulfur atom, i.e. these compounds exist as two optical isomers (enantiomers), or if they also contain one or more asymmetric carbon atoms the compounds have two or more diastereomeric forms, each existing in two enantiomeric forms. Such asymmetric carbon atoms may be the carbon atom on which $R^5$ is attached (when $R^5$ is other than H) or a carbon atom in a substituent.

Both the pure enantiomers, racemic mixtures (50% of each enantiomer) and unequal mixtures of the two are within the scope of the present invention. It should be understood that all the diastereomeric forms possible (pure enantiomers or racemic mixtures) are within the scope of the invention.

The compounds of the invention that are sulfides (X=S) may be asymmetric due to one or more asymmetric carbon atoms, as described above. The different diastereomeric forms possible as well as pure enantiomers and racemic mixtures are within the scope of the invention.

It should be noted that for all the compounds of the invention the substituents $R^1$ and $R^4$ as well as $R^2$ and $R^3$ are considered to be equivalent. This is due to the tautomerism in the imidazole part of the benzimidazole nucleus causing an equilibrium between the two possible >NH-forms. This is illustrated by the following example:

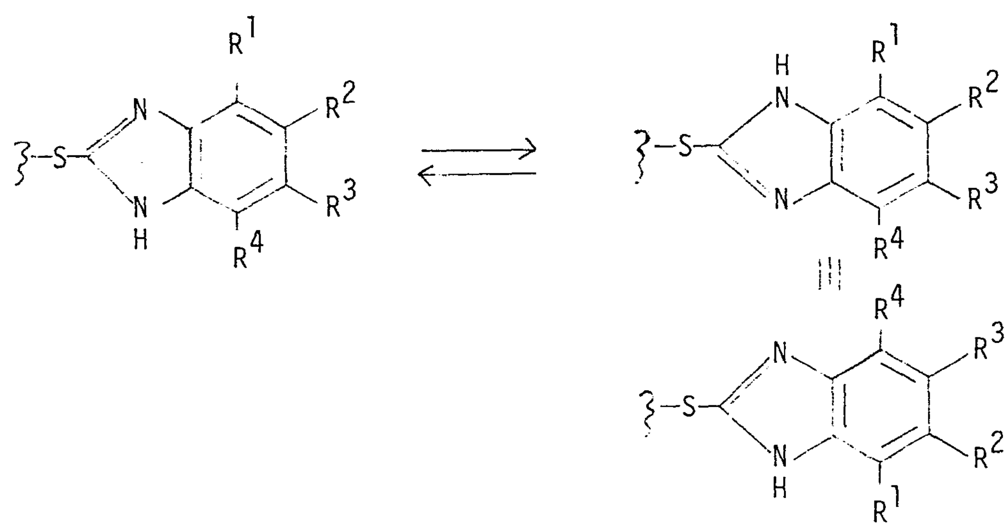

Preferred radicals $R^1$, $R^2$, $R^3$ and $R^4$ are H, $CH_3$, $C_2H_5$, $OCH_3$ and $CF_3$.

Preferred radicals $R^6$ and $R^8$ are H, $CH_3$ and $C_2H_5$.

Preferred meanings of the radical $R^7$ are 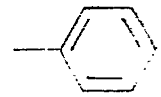 , 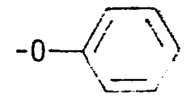 ,

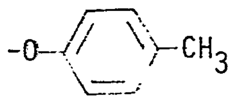 , 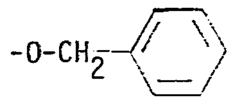 , and 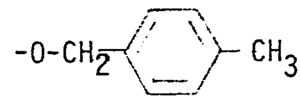 .

The radical $R^5$ is preferably H or $CH_3$.

The radical X is preferably -S-.

The radical X is preferably -SO-.

Particularly preferred radicals are:

| | | |
|---|---|---|
| $R^1$, $R^3$ and $R^4$ | : | H |
| $R^2$ | : | $CH_3$ and $OCH_3$ |
| $R^5$ | : | H |
| $R^6$ and $R^8$ | : | H and $CH_3$, whereby one of $R^6$ and $R^8$ is H |
| $R^7$ | : | 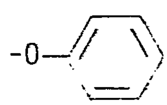 , 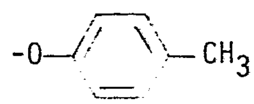 and 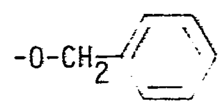 . |

In a preferred embodiment, two or three of the radicals $R^1$, $R^3$ and $R^4$ are H.

In a preferred embodiment, the radical $R^2$ is other than H.

In a preferred embodiment, m is 1 and n is 0.

In a preferred embodiment, m is 1, n is 0, and $R^8$ is $CH_3$ or $C_2H_5$.

In a preferred embodiment, m is 1, n is 0, $R^6$ is H, and $R^8$ is $CH_3$ or $C_2H_5$.

In a preferred embodiment, m is 1, n is 0, $R^6$ is $CH_3$, and $R^8$ is H.

Preferred substitution patterns in the benzimidazole part in the formula I are:

| $R^1$ | $R^2$ | $R^3$ | $R^4$ |
|---|---|---|---|
| H | $CH_3$ | H | H |
| H | $OCH_3$ | H | H |
| H | $CH_3$ | $CH_3$ | H |
| H | H | H | H |
| H | 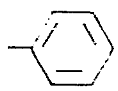 | H | H |

Particularly preferred substitution patterns in the said benzimidazole part are:

| $R^1$ | $R^2$ | $R^3$ | $R^4$ |
|---|---|---|---|
| H | $CH_3$ | H | H |
| H | $OCH_3$ | H | H |
| H | H | H | H |

Preferred substitution patterns in the pyridyl part in the formula I are:

| $R^6$ | $R^7$ | $R^8$ |
|---|---|---|
| H | 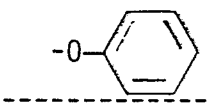 | H |
| H | 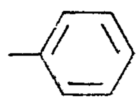 | H |
| H | 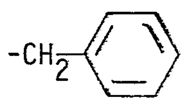 | H |
| H | 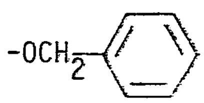 | H |
| H | 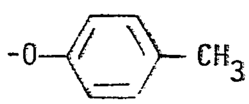 | H |
| $CH_3$ | 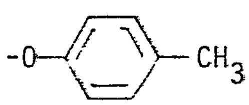 | H |
| H | 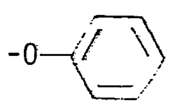 | $CH_3$ |
| H | 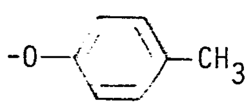 | $CH_3$ |
| H | 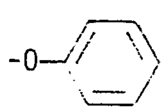 | $C_2H_5$ |
| H | 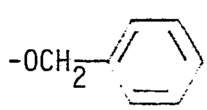 | $CH_3$ |
| H | 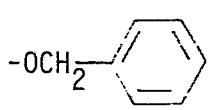 | $C_2H_5$ |

Particularly preferred substitution patterns in the said pyridyl part are:

| $R^6$ | $R^7$ | $R^8$ |
|---|---|---|
| H | 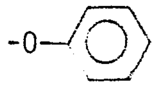 | $C_2H_5$ |
| H | 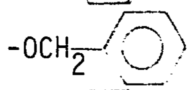 | $CH_3$ |
| $CH_3$ | 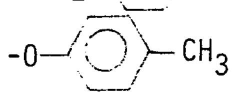 | H |
| H | 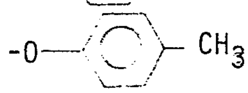 | $CH_3$ |
| H | 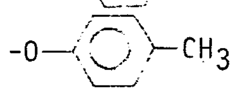 | H |
| H | 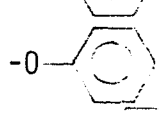 | H |
| H | 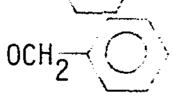 | $C_2H_5$ |

Preferred compounds of the invention are those of the formula

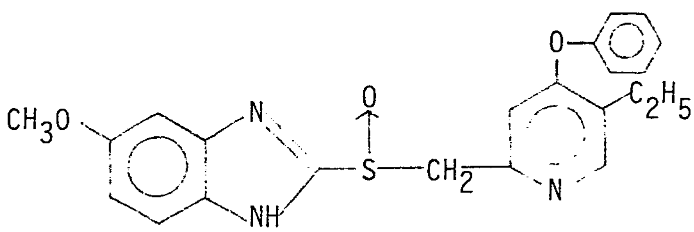

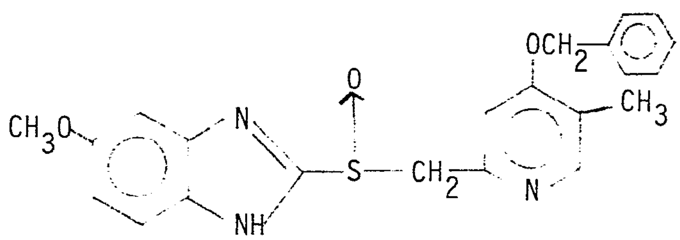

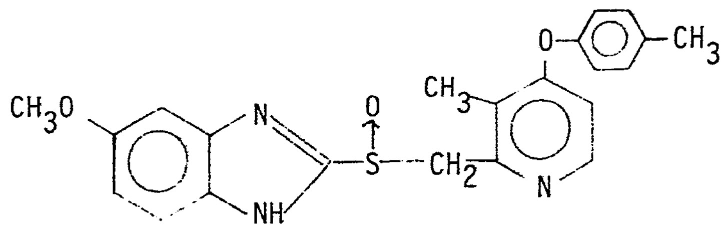

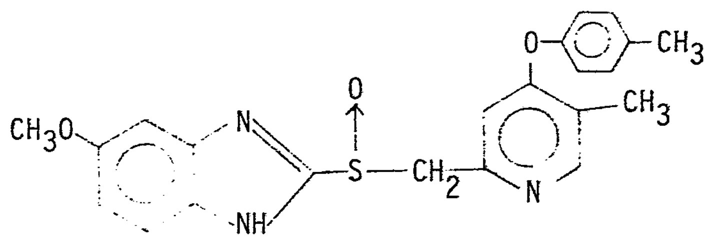

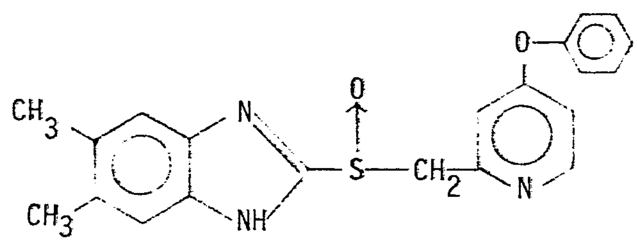

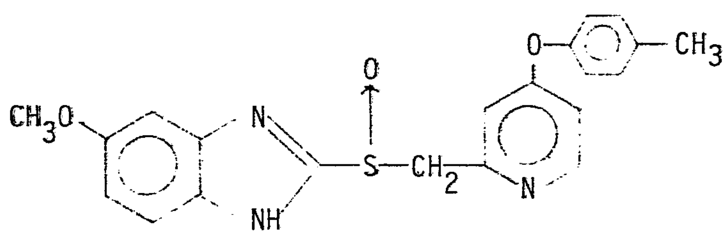

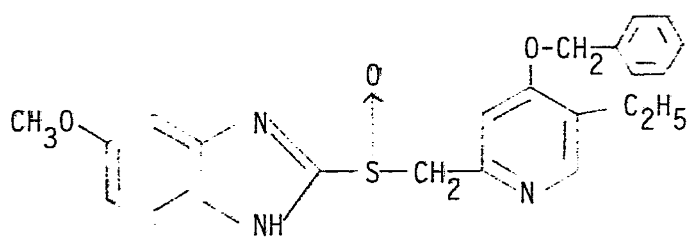

Illustrative examples of compounds included in the scope of the invention are given in the following Table 1.

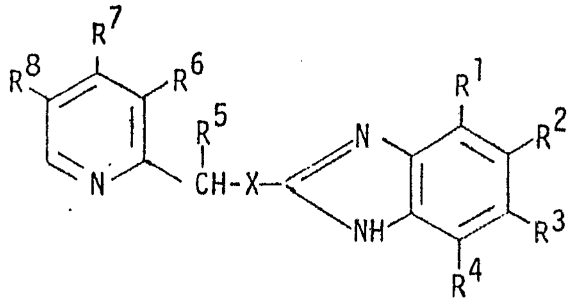

Table 1

Illustrative examples of compounds included in the scope of the invention.

| X | $R^1$ | $R^2$ | $R^3$ | $R^4$ | $R^5$ | $R^6$ | $R^7$ | $R^8$ |
|---|---|---|---|---|---|---|---|---|
| S | $CH_3$ | $CH_3$ | $CH_3$ | $CH_3$ | H | H | 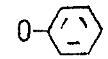 | H |
| SO | $CH_3$ | $CH_3$ | $CH_3$ | $CH_3$ | H | H | 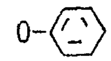 | H |
| S | $CH_3$ | $CH_3$ | $CH_3$ | $CH_3$ | H | H | 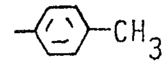 | H |
| SO | $CH_3$ | $CH_3$ | $CH_3$ | $CH_3$ | H | H | 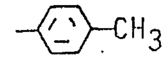 | H |
| S | $CH_3$ | $CH_3$ | $CH_3$ | H | H | H | 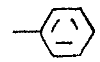 | H |
| SO | $CH_3$ | $CH_3$ | $CH_3$ | H | H | H | 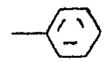 | H |
| S | $CH_3$ | $CH_3$ | $CH_3$ | H | H | H | 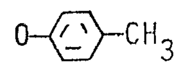 | H |
| SO | $CH_3$ | $CH_3$ | $CH_3$ | H | H | H | 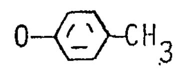 | H |
| S | $CH_3$ | $CH_3$ | H | $CH_3$ | H | H | 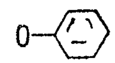 | H |
| SO | $CH_3$ | $CH_3$ | H | $CH_3$ | H | H | 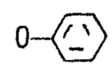 | H |
| S | $CH_3$ | $CH_3$ | H | $CH_3$ | H | H | 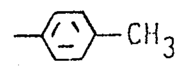 | H |
| SO | $CH_3$ | $CH_3$ | H | $CH_3$ | H | H | 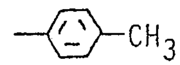 | H |
| S | $CH_3$ | $CH_3$ | H | H | H | H | 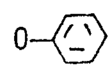 | H |
| S | H | H | H | H | H | H | 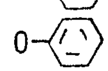 | $C_2H_5$ |
| SO | H | H | H | H | H | H | 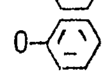 | $C_2H_5$ |

cont.

| X | $R^1$ | $R^2$ | $R^3$ | $R^4$ | $R^5$ | $R^6$ | $R^7$ | $R^8$ |
|---|---|---|---|---|---|---|---|---|
| SO | $CH_3$ | $CH_3$ | H | H | H | H | 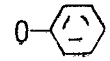 | H |
| S | H | $CH_3$ | $CH_3$ | H | H | H | 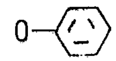 | $CH_3$ |
| SO | H | $CH_3$ | $CH_3$ | H | H | H | 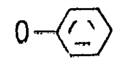 | $CH_3$ |
| S | $CH_3$ | H | H | $CH_3$ | H | H | 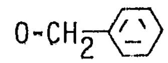 | H |
| SO | $CH_3$ | H | H | $CH_3$ | H | H | 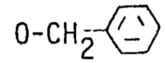 | H |
| S | $CH_3$ | H | H | H | H | $CH_3$ | 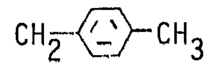 | $CH_3$ |
| SO | $CH_3$ | H | H | H | H | $CH_3$ | 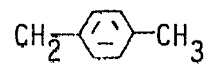 | $CH_3$ |
| S | H | $CH_3$ | H | H | H | $CH_3$ | 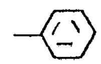 | H |
| SO | H | $CH_3$ | H | H | H | $CH_3$ | 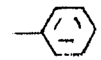 | H |
| S | H | $OCH_3$ | H | H | H | H | 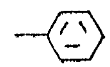 | H |
| SO | H | $OCH_3$ | H | H | H | H | 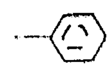 | H |
| S | H | $OCH_3$ | H | H | H | H | 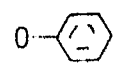 | H |
| SO | H | $OCH_3$ | H | H | H | H | 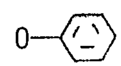 | H |
| S | H | $OCH_3$ | H | H | H | H | 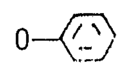 | $CH_3$ |
| SO | H | $OCH_3$ | H | H | H | H | 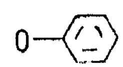 | $CH_3$ |
| S | H | $OCH_3$ | H | H | H | H | 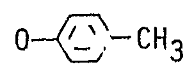 | $CH_3$ |
| SO | H | $OCH_3$ | H | H | H | H | 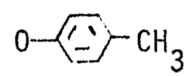 | $CH_3$ |
| S | H | $OCH_3$ | H | H | H | H | 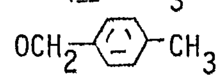 | $C_2H_5$ |
| SO | H | $OCH_3$ | H | H | H | H | 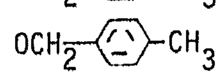 | $C_2H_5$ |

cont.

| X | $R^1$ | $R^2$ | $R^3$ | $R^4$ | $R^5$ | $R^6$ | $R^7$ | $R^8$ |
|---|---|---|---|---|---|---|---|---|
| S | H | $OCH_3$ | H | H | H | H | 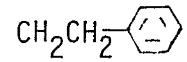 | H |
| SO | H | $OCH_3$ | H | H | H | H | 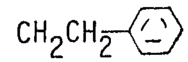 | H |
| S | H | $OCH_3$ | H | H | H | H | 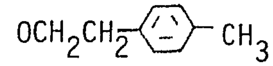 | H |
| SO | H | $OCH_3$ | H | H | H | H | 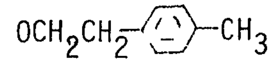 | H |
| S | H | $OCH_3$ | H | H | H | $CH_3$ | 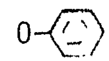 | H |
| SO | H | $OCH_3$ | H | H | H | $CH_3$ | 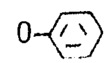 | H |
| S | H | $OCH_3$ | H | H | H | $CH_3$ | 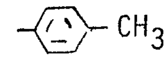 | H |
| SO | H | $OCH_3$ | H | H | H | $CH_3$ | 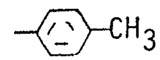 | H |
| S | H | $OCH_3$ | H | H | H | $C_2H_5$ | 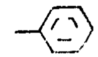 | H |
| SO | H | $OCH_3$ | H | H | H | $C_2H_5$ | 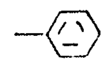 | H |
| S | H | $OCH_3$ | H | H | H | $C_2H_5$ | 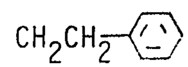 | H |
| SO | H | $OCH_3$ | H | H | H | $C_2H_5$ | 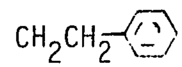 | H |
| S | H | $OCH_3$ | H | H | H | $C_2H_5$ | 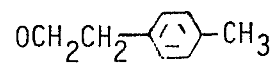 | H |
| SO | H | $OCH_3$ | H | H | H | $C_2H_5$ | 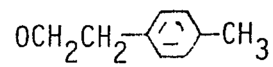 | H |
| S | H | $OCH_3$ | H | H | H | $CH_3$ | 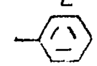 | $CH_3$ |
| SO | H | $OCH_3$ | H | H | H | $CH_3$ | 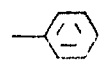 | $CH_3$ |
| S | H | $OCH_3$ | H | H | H | $CH_3$ | 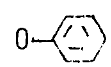 | $CH_3$ |
| SO | H | $OCH_3$ | H | H | H | $CH_3$ | 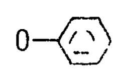 | $CH_3$ |
| S | H | $OCH_3$ | H | H | H | $CH_3$ | 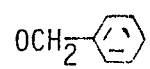 | $CH_3$ |

cont.

| X | $R^1$ | $R^2$ | $R^3$ | $R^4$ | $R^5$ | $R^6$ | $R^7$ | $R^8$ |
|---|---|---|---|---|---|---|---|---|
| SO | H | $OCH_3$ | H | H | H | $CH_3$ | 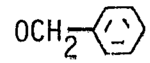 | $CH_3$ |
| S | $CH_3$ | $OCH_3$ | $CH_3$ | H | H | H | 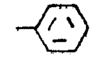 | H |
| SO | $CH_3$ | $OCH_3$ | $CH_3$ | H | H | H | 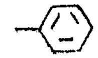 | H |
| S | $CH_3$ | $OCH_3$ | $CH_3$ | H | H | H | 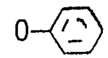 | $CH_3$ |
| SO | $CH_3$ | $OCH_3$ | $CH_3$ | H | H | H | 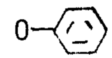 | $CH_3$ |
| S | H | $CH_3$ | H | H | H | H | 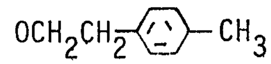 | H |
| SO | H | $CH_3$ | H | H | H | H | 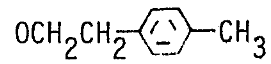 | H |
| S | H | $CH_3$ | H | H | H | $CH_3$ | 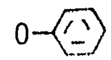 | H |
| SO | H | $CH_3$ | H | H | H | $CH_3$ | 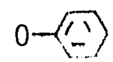 | H |
| S | H | $CH_3$ | H | H | H | $CH_3$ | 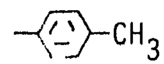 | H |
| SO | H | $CH_3$ | H | H | H | $CH_3$ | 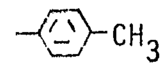 | H |
| S | H | $CH_3$ | H | H | H | $C_2H_5$ | 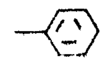 | H |
| SO | H | $CH_3$ | H | H | H | $C_2H_5$ | 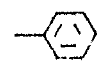 | H |
| S | H | $CH_3$ | H | H | H | $C_2H_5$ | 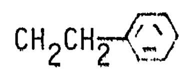 | H |
| SO | H | $CH_3$ | H | H | H | $C_2H_5$ | 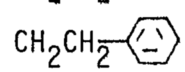 | H |
| S | H | $CH_3$ | H | H | H | $C_2H_5$ | 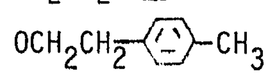 | H |
| SO | H | $CH_3$ | H | H | H | $C_2H_5$ | 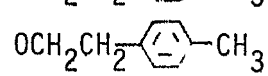 | H |
| S | H | $CH_3$ | H | H | H | $CH_3$ | 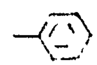 | $CH_3$ |
| SO | H | $CH_3$ | H | H | H | $CH_3$ | 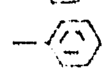 | $CH_3$ |

cont.

| X | $R^1$ | $R^2$ | $R^3$ | $R^4$ | $R^5$ | $R^6$ | $R^7$ | $R^8$ |
|---|---|---|---|---|---|---|---|---|
| S | H | $CH_3$ | H | H | H | $CH_3$ | O 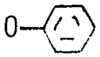 | $CH_3$ |
| SO | H | $CH_3$ | H | H | H | $CH_3$ | O 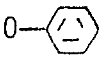 | $CH_3$ |
| S | H | $CH_3$ | H | H | H | $CH_3$ | $OCH_2$ 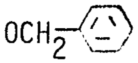 | $CH_3$ |
| SO | H | $CH_3$ | H | H | H | $CH_3$ | $OCH_2$ 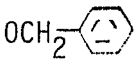 | $CH_3$ |
| S | $CH_3$ | $CH_3$ | $CH_3$ | H | H | H | 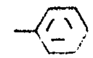 | H |
| SO | $CH_3$ | $CH_3$ | $CH_3$ | H | H | H | 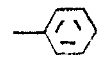 | H |
| S | $CH_3$ | $CH_3$ | $CH_3$ | H | H | H | O 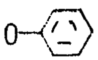 | $CH_3$ |
| SO | $CH_3$ | $CH_3$ | $CH_3$ | H | H | H | O 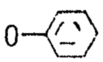 | $CH_3$ |
| SO | H | $CF_3$ | H | H | H | H | 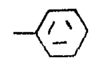 | H |
| S | H | $CF_3$ | H | H | H | H | 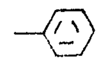 | H |
| SO | H | $CF_3$ | H | H | H | H | O 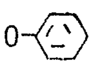 | $CH_3$ |
| S | H | $CF_3$ | H | H | H | H | O 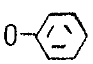 | $CH_3$ |
| SO | H | Cl | H | H | H | H | O 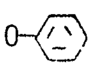 | H |
| S | H | Cl | H | H | H | H | O 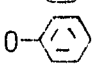 | H |
| SO | H | Cl | H | H | H | H | $OCH_2CH_2$ 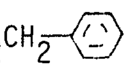 | $CH_3$ |
| S | H | Br | H | H | H | H | 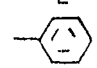 | $CH_3$ |
| SO | H | Br | H | H | H | $CH_3$ | 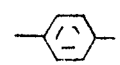 $CH_3$ | H |
| S | H | Br | H | H | H | $CH_3$ | $OCH_2CH_2$ 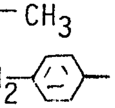 $CH_3$ | H |
| SO | H | F | H | H | H | H | $OCH_2CH_2$ 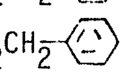 | H |

cont.

| X | $R^1$ | $R^2$ | $R^3$ | $R^4$ | $R^5$ | $R^6$ | $R^7$ | $R^8$ |
|---|---|---|---|---|---|---|---|---|
| S | H | 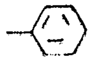 | H | H | H | H | 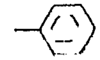 | H |
| SO | H | 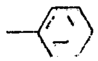 | H | H | H | H | 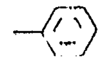 | H |
| S | H | 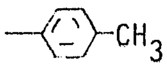 | H | H | H | H | 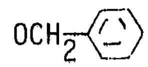 | H |
| SO | H | 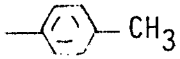 | H | H | H | H | 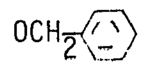 | H |
| S | H | $COCH_3$ | H | H | H | $CH_3$ | 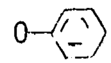 | H |
| SO | H | $COCH_3$ | H | H | H | $CH_3$ | 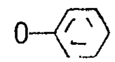 | H |
| S | H | $OCH_2CH_2OCH_3$ | H | H | H | H | 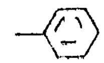 | $CH_3$ |
| SO | H | $OCH_2CH_2OCH_3$ | H | H | H | H | 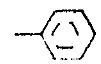 | $CH_3$ |
| S | $CH_3$ | $COCH_3$ | $CH_3$ | H | H | H | 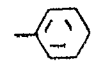 | H |
| SO | $C_2H_5$ | $COCH_3$ | $CH_3$ | H | H | H | 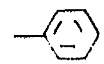 | H |
| S | H | 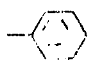 | H | H | H | H | 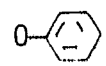 | H |
| SO | H | 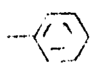 | H | H | H | H | 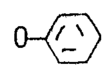 | H |
| S | H | 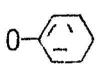 | H | H | H | H | 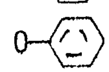 | H |
| SO | H | 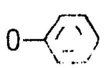 | H | H | H | H | 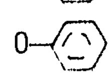 | H |
| S | H | 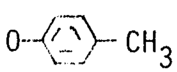 | H | H | H | H | 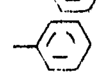 | H |
| SO | H | 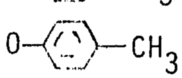 | H | H | H | H | 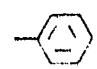 | H |
| S | H | 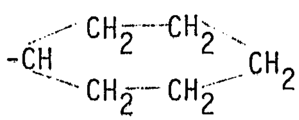 | H | H | H | H | 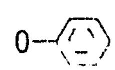 | $C_2H_5$ |
| SO | H | 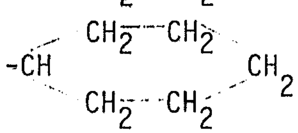 | H | H | H | H | 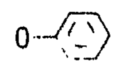 | $C_2H_5$ |

The invention takes into consideration that compounds that structurally deviate from the formula I, after administration to a living organism may be transformed to a compound of formula I and in this structural form exert their effect. Such compounds structurally deviating from compounds of the formula I, are included in the scope of the invention.

Likewise, certain compounds of formula I may be metabolized into other compounds of formula I before exerting their effect. Compounds of the invention wherein X is S are thus believed to exert their antisecretory and cytoprotective activities after metabolism to compounds wherein X is SO. These considerations are also a further aspect of the invention.

Further, it is believed that all compounds of formula I wherein X is SO after administration to a living organism, exert their antisecretory and cytoprotective effects after metabolic or pure chemical transformation to another, reactive species. Accordingly, the same is true also for the compounds of formula I wherein X is S, but via initial transformation to the corresponding compounds of formula I wherein X is SO. These considerations as well as such reactive species per se are included within the scope of the present invention.

## Preparation

Compounds of formula I above may be prepared according to the following methods:

a) Oxidizing a compound of the formula I,

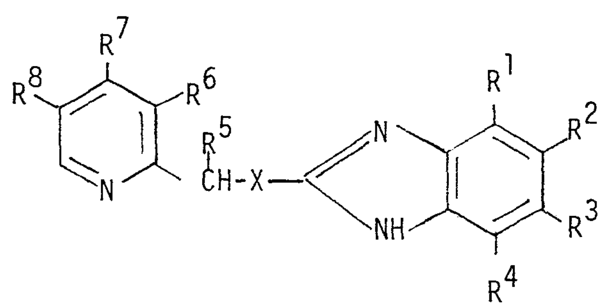

I

wherein X is S and $R^1$, $R^2$, $R^3$, $R^4$, $R^5$, $R^6$, $R^7$ and $R^8$ have the meanings given, to give a compound of the same formula I wherein X is SO. This

oxidation may be carried out by using an oxidizing agent selected from the group consisting of nitric acid, hydrogen peroxide, peracids, peresters, ozone, dinitrogentetraoxide, iodosobenzene, N-halosuccinimide, 1-chlorobenzotriazole, t-butylhypochlorite, diazabicyclo-[2,2,2]-octane bromine complex, sodium metaperiodate, selenium dioxide, manganese dioxide, chromic acid, cericammonium nitrate, bromine, chlorine, and sulfuryl chloride. The oxidation usually takes place in a solvent wherein the oxidizing agent is present in some excess in relation to the product to be oxidized.

The oxidation may also be carried out enzymatically by using an oxidating enzyme or microbiotically by using a suitable microorganism.

b) Reacting a compound of the formula

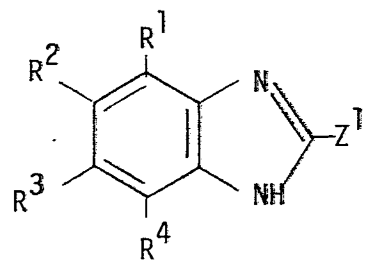

II

with a compound of the formula

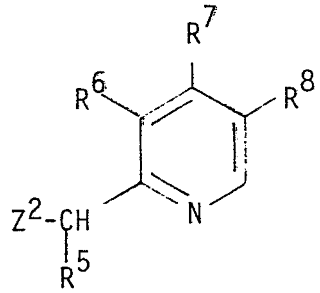

III

in which formulas $R^1$, $R^2$, $R^3$, $R^4$, $R^5$, $R^6$, $R^7$ and $R^8$ are as defined previously and wherein one of $Z^1$ and $Z^2$ is SH and the other is a leaving group, gives a compound of the formula I wherein X is S.

Examples of leaving groups $Z^1$ and $Z^2$ in the compounds II and III are halogens, preferably chlorine, bromine or iodine, acyloxy radicals, for example residues of strong organic sulfonic acids, for instance of an arylsulfonic acid, for example tosyloxy or an alkylsulfonic acid, for example mesyloxy, alkylmercapto groups, for example methylmercapto, alkylsulfinyl groups, for example methylsulfinyl and the like.

Thus, $Z^1$ or $Z^2$ when designating leaving groups may be a reactive esterified hydroxy group. The esterification may be carried out with an organic acid or with an inorganic acid such as HCl, HBr or $H_2SO_4$.

The reaction of a compound of formula II above with a compound of formula III is conveniently carried out in the presence of a suitable solvent that is inert under the reaction conditions utilized as described herinafter. The reaction may further be carried out in the presence of a suitable base. Suitable bases include, for example, inorganic bases such as sodium or potassium hydroxide, sodium or potassium alkoxide, sodium or potassium hydride and the like, organic bases such as tertiary amines, for example triethylamine and the like.

Suitable solvents for the above described reaction include, for example, alcohols, preferably lower alkanols such as methanol and ethanol, mixtures of such alcohols with water, ethers, such as tetrahydrofuran, halogenated hydrocarbons, such as methylene chloride. Aprotic solvents such as ethers and halogenated hydrocarbons are necessary in the case of sodium and potassium hydride.

The reaction of the compounds of formulas II and III may be carried out at a temperature between the ambient temperature and the boiling temperature of the reaction mixture. It is preferred to carry out the reaction, however, at a temperature at or close to the boiling point of the reaction mixture for the preparation of a compound of the formula I wherein $R^5$ is H.

c) Hydrolyzing a compound of the formula

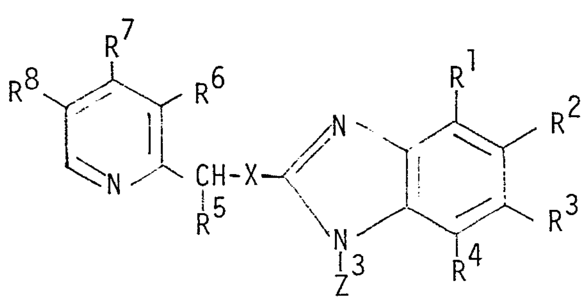

IV

wherein X, $R^1$, $R^2$, $R^3$, $R^4$, $R^6$, $R^7$ and $R^8$ are as defined above and $Z^3$ is a suitable N-protecting group such as alkanoyl, carboalkoxy and trimethylsilyl, to the formation of a compound of the formula I.

The alkanoyl group in $Z^3$ can have 1-6 carbon atoms and the carboalkoxy group 2-6 carbon atoms. The hydrolysis may be performed in alkaline solution or in acidic solution, the latter mainly for compounds wherein X is S.

d) Reacting a compound of the formula

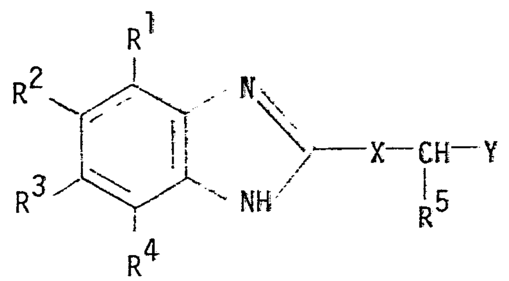

V

wherein $R^1$, $R^2$, $R^3$, $R^4$, $R^5$ and X are as defined above and Y is K, Na or Li,

with a compound of the formula

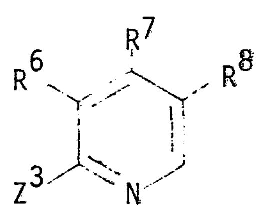

VI

wherein $R^6$, $R^7$ and $R^8$ are as defined above and $Z^3$ is halogen such as Cl or Br;

whereafter the compound of the formula I obtained if desired, when X is -S-, is converted to a physiologically acceptable salt or oxidized to form a compound of the formula I wherein X is -SO-.

Depending on the process conditions and the starting materials, the end products of the formula I wherein X is S is obtained either as the free base or as a salt. The end products of the formula I wherein X is -SO- are obtained as the free base. Both the free base and the salts

of these end products are included within the scope of the invention. Thus, basic, neutral or mixed salts may be obtained as well as hemi, mono, sesqui or polyhydrates. Acid addition salts of the new sulfides may in a manner known per se be transformed into free base using basic agents such as alkali or by ion exchange. The free bases of the sulfides obtained may also form salts with organic or inorganic acids. In the preparation of acid addition salts preferably such acids are used which form suitable therapeutically acceptable salts.

Example of such acids are hydrohalogen acids, sulfonic acid, phosphoric acid, nitric acid, and perchloric acid; aliphatic, alicyclic, aromatic or heterocyclic carboxyl or sulfonic acids, such as formic acid, acetic acid, propionic acid, succinic acid, glycolic acid, lactic acid, malic acid, tartaric acid, citric acid, ascorbic acid, maleic acid, hydroxymaleic acid, pyruvic acid, phenylacetic acid, benzoic acid, p-aminobenzoic acid, p-hydroxybenzoic acid, salicylic acid or p-aminosalicylic acid, embonic acid, methanesulfonic acid, ethanesulfonic acid, hydroxyethanesulfonic acid, ethylenesulfonic acid, halogenbenzenesulfonic acid, toluenesulfonic acid, naphtylsulfonic acid or sulfanilic acids, methionine, tryptophane, lysine or arginine.

These or other salts of the new sulfide compounds, as e.g. picrates, may serve as purifying agents of the free bases obtained. Salts of the bases may be formed, separated from solution, and then the free base can be recovered in higher purity from a new salt solution.

Also included in the scope of the invention are alkaline salts of the compounds of the formula I wherein X is SO. These alkaline salts are exemplified by the formula

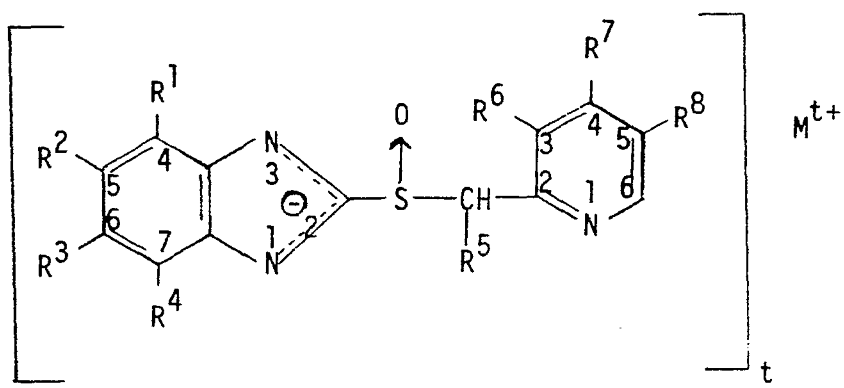

VII

wherein t is 1, 2 or 4; $M^{t+}$ is $Li^+$, $Na^+$, $K^+$, $Mg^{2+}$, $Ca^{2+}$, $Ti^{4+}$, $N^+(R)_4$ or 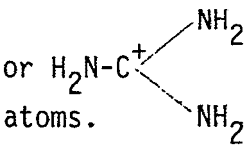, wherein R is an alkyl group containing 1-4 carbon atoms.

Particularly preferred are the $Na^+$, $Ca^{2+}$ and $Mg^{2+}$ salts. The salts of the formula VII exhibit improved stability which means that they can be stored for extended periods of time without substantial degradation.

The alkaline salts of the invention of the formula VII are prepared by reacting a compound of the formula I wherein X is SO:

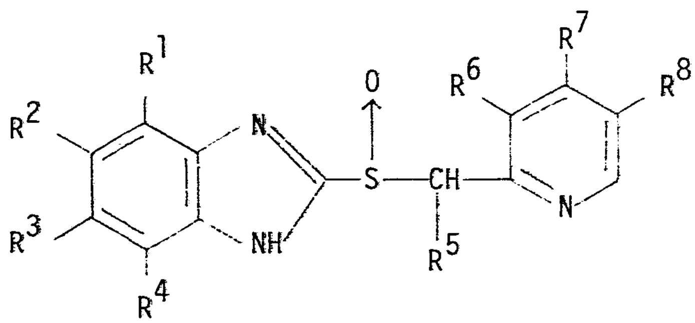

Ia

with a base capable of releasing the cation $M^{t+}$,

wherein $M^{t+}$ is as defined above, to give a salt of the formula VII,

which salt is thereafter isolated.

Examples of bases capable of releasing the cation $M^{t+}$, and examples of reaction conditions are given below.

a) Salts of the formula VII wherein M is Li, Na or K are prepared by treating a compound of the formula Ia with LiOH, NaOH or KOH in an aqueous or nonaqueous medium or with LiOR, $LiNH_2$, $LiNR_2$, NaOR, $NaNH_2$, $NaNR_2$, KOR, $KNH_2$ or $KNR_2$, wherein R is an alkyl group containing 1-4 carbon atoms, in a nonaqueous medium.

b) Salts of the formula VII, wherein M is Mg, Ca, or Ti are prepared by treating a compound of the formula Ia with $Mg(OR)_2$, $Ca(OR)_2$, $CaH_2$, $Ti(OR)_4$ or $TiH_4$, wherein R is an alkyl group containing 1-4 carbon atoms, in a nonaqueous solvent such as an alcohol (only for the alcoholates), e.g. ROH, or in an ether such as tetrahydrofuran.

Salts of the formula VII, wherein M is 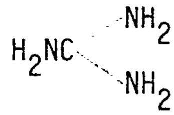 are prepared by treating a compound of the formula Ia with the strong base 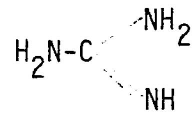 dissolved in a solvent, for example an alcohol.

d) A salt of formula VII may be converted to another salt of the same formula by exchanging the cation. When both the starting material and the salt obtained as final product are sufficiently soluble, such an exchange may be performed by using a cation-exchange resin saturated with the cation desired in the product. The exchange may also be performed by utilizing the low solubility of a desired salt. By this principle, for example, $Na^+$ as a counter ion may be exchanged for $Ca^{2+}$ or $Mg^{2+}$.

e) The reaction between the compound Ia and $M^{t+}$ may also be carried out by ion-pair extraction. For example, tetrabutylammonium salts of the invention may be prepared by dissolving the $Na^+$-salt in water containing tetrabutylammonium sulfate followed by extraction of the tetrabutylammonium salt VII into a methylene chloride phase, and subsequent isolation of the tetrabutylammonium salt. In this manner also other tetraalkylammonium salts may be prepared.

Illustrative examples of the radical R are $CH_3$, $C_2H_5$, n-$C_3H_7$, n-$C_4H_9$, i-$C_4H_9$, sec.-$C_4H_9$ and tert.-$C_4H_9$.

Racemates obtained of compounds of the formula I can be separated according to known methods, e.g. recrystallization from an optically active solvent, use of microorganisms, reactions with optically active acids forming diastereomeric salts which can be separated, (e.g. separation based on different solubilities of the diastereomers), acylation of the benzimidazole nitrogen or another oxygen atom in a substituent by an optically active activated carboxylic acid (e.g. acid chloride), followed by chromatographic separation and deacylation.

Suitable optically active acids for salt formation are the L- and D-forms of tartaric acid, di-o-tolyl-tartaric acid, malic acid, mandelic acid, camphorsulfonic acid or quinic acid, and for acylation O-methylmandelic acid. Preferably the more active part of the two antipodes is isolated.

In the case of diastereomeric mixtures (racemate mixtures) these may be separated into stereoisomeric (diastereomeric) pure racemates by means of chromatography or fractional crystallization.

The starting materials utilized in the processes a and c may be obtained via process b. The starting materials used for process b are in some cases known, but may in some cases not be known. These novel starting materials may, however, be obtained according to processes known per se.

Starting materials of the formula II

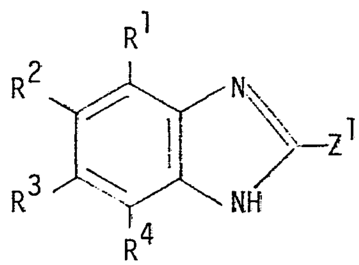

II

wherein $Z^1$ is SH may be obtained from the corresponding o-phenylenediamine by reaction with potassium ethylxanthate (Org. Synth. Vol. 30, p. 56) or thiophosgene.

The compounds of the formula II wherein $Z^1$ is alkylmercapto and alkylsulfinyl may be obtained from the above-mentioned compound by simple S-alkylation with alkyl halide and by oxidation of the product from the S-alkylation, respectively.

The compounds of the formula II wherein $Z^1$ is halogen or acyloxy may be obtained from compounds of the same formula wherein $Z^1$ is OH by treatment with $POCl_3$, $POBr_3$ and the like or the appropriate acyl halide, respectively. The starting material wherein $Z^1$ is OH is obtained from the corresponding o-phenylenediamine by reaction with phosgene.

The o-phenylenedeamines required may be obtained from the corresponding substituted benzenes according to processes known per se, e.g. by the consecutive processes: nitration, reduction, acetylation, nitration, deacetylation and reduction, or from one of the intermediary stages just mentioned.

Starting materials of the formula

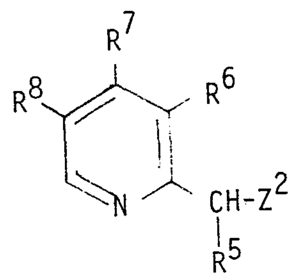

III

wherein $R^5$ is H, may be obtained either from the correspondingly substituted ($R^6$, $R^7$ and $R^8$) 2-methyl-substituted pyridine N-oxide via a known rearrangement to the intermediate 2-pyridinylmethanol or via a hydroxymethylation of the substituted ($R^6$, $R^7$ and $R^8$) pyridine to give the same intermediate, and then treatment of the 2-pyridinylmethanol with halogenating agents such as thionyl chloride or O-acylating agents such as p-toluenesulfonyl chloride to give compounds of the formula III wherein $Z^2$ is halogen and sulfonyloxy groups, respectively.

These leaving groups may then be substituted for alkylmercapto groups by treatment with e.g. sodium alkylmercaptide, which may then be oxidized to an alkylsulfinyl group, or substituted for SH by treatment with e.g. NaSH.

For the preparation of intermediates of formula

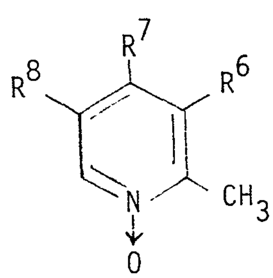

VIII

wherein $R^6$ and $R^8$ are as defined above, and wherein m in the radical $R^7$ is 1, a compound of formula VI, wherein $R^7$ is $NO_2$, is reacted by the corresponding sodium aryloxide.

The starting materials of the formula

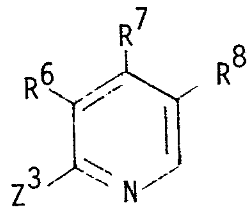

VI

may be prepared by reacting the corresponding 2-hydroxypyridine with a halogenating agent such as phosphoroxychloride or phosphoroxybromide.

The intermediates of the formulas III and VI are included in the scope of the invention.

The invention also relates to pharmaceutical compositions containing the compound with the formula I or its salts as active ingredient; to the use of the novel compounds for providing local gastrointestinal cytoprotective effects in mammals and man; to the use of the novel compounds in the prevention and treatment of gastrointestinal inflammatory diseases in mammals and man; to the use of the novel compounds for inhibiting gastric acid secretion in mammals and man; to a method for inhibiting gastric acid secretion in mammals and man by administering a compound of the formula I or its salt; to a method for the treatment of gastrointestinal inflammatory diseases in mammals and man by administering a compound of the formula I or its salt; and to a method for providing gastrointestinal cytoprotective effects in mammals and man by orally administering a compound of the formula I or its salt.

For clinical use the compounds of the invention are formulated into pharmaceutical formulations for oral, rectal, parenteral or other mode of administration. The pharmaceutical formulation contains a compound of the invention in combination with a pharmaceutically acceptable carrier. The carrier may be in the form of a solid, semi-solid or liquid diluent, or a capsule. These pharmaceutical preparations are a further object of the invention. Usually the amount of active compounds is between 0.1-95% by weight of the preparation, between 0.2-20% by weight in preparations for parenteral use and between 1 and 50% by weight in preparations for oral administration.

In the preparation of pharmaceutical formulations containing a compound of the present invention in the form of dosage units for oral administration the compound selected may be mixed with a solid, powdered carrier, such as lactose, saccharose, sorbitol, mannitol, starch, amylopectin, cellulose derivatives, gelatine, or another suitable carrier, as well as with lubricating agents such as magnesium stearate, calcium stearate, sodium steryl fumarate and polyethylene glycol waxes. The mixture is then processed into granules or pressed into tablets. Since the sulfoxides of the invention are susceptible to degradation in acid to neutral media, granules and tablets containing sulfoxides are preferably coated with an enteric coating which protects the active compound from acid degradation as long as the dosage form remains in the stomach. The enteric coating is chosen among pharmaceutically acceptable enteric-coating materials e.g. beeswax, shellac or anionic film-forming polymers such as cellulose acetate phthalate, hydroxypropylmethylcellulose phthalate, partly methyl esterified methacrylic acid polymers and the like, if preferred in combination with a suitable plasticizer. To this coating various dyes may be added in order to distinguish among tablets or granules with different active compounds or with different amounts of the active compound present.

Soft gelatine capsules may be prepared with capsules containing a mixture of the active compound or compounds of the invention, vegetable oil, fat, or other suitable vehicle for soft gelatine capsules. Soft gelatine capsules may also be enteric coated as described above. Hard gelatine capsules may contain granules or enteric-coated granules of the active compound. Hard gelatine capsules may also contain the active compound in combination with a solid powdered carrier such as lactose, saccharose, sorbitol, mannitol, potato starch, corn starch, amylopectin, cellulose derivatives or gelatine. The hard gelatine capsules may be enteric coated as described above.

Dosage units for rectal administration may be prepared in the form of suppositories which contain the active substance mixed with a neutral fat base, or they may be prepared in the form of a gelatine rectal capsule which contains the active substance in a mixture with a vegetable oil, paraffin oil or other suitable vehicle for gelatine rectal capsules, or they may be prepared in the form of a ready-made micro enema, or they may be prepared in the form of a dry micro enema formulation to be reconstituted in a suitable solvent just prior to administration.

Liquid preparations for oral administration may be prepared in the form of syrups or suspensions, e.g. solutions or suspensions containing from 0.2% to 20% by weight of the active ingredient and the remainder consisting of sugar or sugaralcohols and a mixture of ethanol, water, glycerol, propylene glycol and polyethylene glycol. If desired, such liquid preparations may contain colouring agents, flavouring agents, saccharine and carboxymethyl cellulose or other thickening agent. Liquid preparations for oral administration may also be prepared in the form of a dry powder to be reconstituted with a suitable solvent prior to use.

Solutions for parenteral administration may be prepared as a solution of a compound of the invention in a pharmaceutically acceptable solvent, preferably in a concentration from 0.1% to 10% by weight. These solutions may also contain stabilizing agents and/or buffering agents and may be manufactured in different unit dose ampoules or vials. Solutions for parenteral administration may also be prepared as a dry preparation to be reconstituted with a suitable solvent extemporaneously before use. Suitably the sodium salt of a compound of the invention is used.

Special precautions may have to be taken with pharmaceutical preparations intended for oral use in order to obtain a gastric acid inhibiting effect. It may be necessary to manufacture sustained release preparations in order that the desired gastric acid inhibiting effect will be obtained despite possible degradation of the administered substance of the formula I in the gastric juice.

Oral administration will be suitable for obtaining gastrointestinal protective effects. Formulations suitable for cytoprotection are preferably non-enteric coated oral dosage forms. Oral preparations for gastric acid inhibition are preferably enteric coated dosage forms.

The typical daily dose of the active substance varies within a wide range and will depend on various factors such as for example the individual requirement of each patient, the route of administration and the disease. In general, oral and parenteral dosages will be in the range of 5 to 500 mg per day of active substance.

The invention is illustrated by the following examples.

Example 1. Method a. Preparation of 5,6-dimethyl-2-[[(4-phenoxy-5--ethyl-2-pyridinyl)methyl]sulfinyl]-1H-benzimidazole.

m-Chloroperbenzoic acid, 90.4% (0.56 g, 0.0029 mol) dissolved in $CH_2Cl_2$ (15 ml) was added under stirring to a cooled (-2°C) solution of 5,6-dimethyl-2[[(4-phenoxy-5-ethyl-2-pyridinyl)methyl]thio]-1H-benzimidazole (1.2 g, 0.0029 mol) in $CH_2Cl_2$ (50 ml) maintaining the temperature at -2°C. Stirring was continued at -2°C for 5 min and then $NaHCO_3$ (0.75 g, 0.0089 mol) dissolved in water (50 ml) was added under vigorous stirring. The two phases were separated and the $CH_2Cl_2$-phase was washed with water (20 ml). The organic phase was dried ($MgSO_4$) and the solvent was evaporated off. The residue was crystallized from $CH_3CN$ yielding the desired product (0.68 g, 57%), m.p. 146°C.

Example 2. Method b. Preparation of 5,6-dimethyl-2-[[(4-phenyl-2--pyridinyl)methyl]thio]-1H-benzimidazole.

To 5,6-dimethyl-2-mercapto-1H-benzimidazole (2.23 g, 0.0125 mol) in methanol (50 ml) were added (in the following order) NaOH (0.5 g, 0.0125 mol) dissolved in water (1.5 ml) and 4-phenyl-2-chloromethylpyridine hydrochloride (3.0 g, 0.0125 mol). The mixture was heated until reflux. NaOH (0.5 g, 0.0125 mol) dissolved in water (1.5 ml) was added dropwise and then the reflux was continued for 3 hours. The mixture was poured on water (30 ml) and extracted with $CH_2Cl_2$ (30 ml). The organic phase was dried ($Na_2SO_4$) and the solvent evaporated off. The residue was crystallized from diethylether giving the desired product (3.25 g, 75%), m.p. 120°C.

Example 3. Method a. Preparation of 5-methoxy-2-[[[4-(4-methylphenoxy)--2-pyridinyl]methyl]sulfinyl]-1H-benzimidazole.

m-Chloroperbenzoic acid, 82% (1.54 g, 0.0073 mol) dissolved in $CH_2Cl_2$ (20 ml) was added under stirring to a cooled (-5°C) solution of 5--methoxy-2-[[[4-(4-methylphenoxy)-2-pyridinyl]methyl]thio]-1H-benzimidazole (2.9 g, 0.0073 mol) in $CH_2Cl_2$ (50 ml) maintaining the temperature at -5°C. After the addition NaOH (0.87 g, 0.022 mol) dissolved in water

(30 ml) was added under vigorous stirring. The two phases were separated. More $CH_2Cl_2$ (40 ml) was added to the aqueous phase, the pH was adjusted to 9.5 by adding 2M HCl and after stirring the phases were separated. The organic phase was dried ($Na_2SO_4$) and the solvent was evaporated off. The residue was crystallized from $CH_3CN$ yielding the desired product (1.2 g, 42%), m.p. 121°C.

The following Table 2 gives data for examples 1-43 of compounds of the invention.

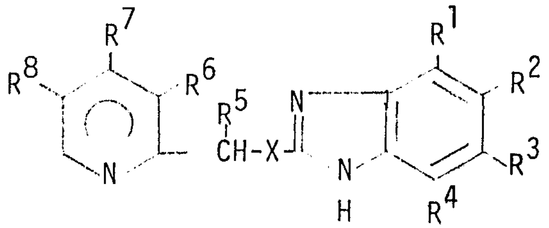

**Table 2** Summary of working examples

| Ex | X | $R^1$ | $R^2$ | $R^3$ | $R^4$ | $R^5$ | $R^6$ | $R^7$ | $R^8$ | Method (Ex No) | Yield | M.p. (°C) other data |
|---|---|---|---|---|---|---|---|---|---|---|---|---|
| 1 | SO | H | $CH_3$ | $CH_3$ | H | H | H | 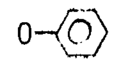 | $C_2H_5$ | a(Ex 1) | 57 | 146 |
| 2 | S | H | $CH_3$ | $CH_3$ | H | H | H | 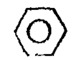 | H | b(Ex 2) | 75 | 120 |
| 3 | SO | H | $OCH_3$ | H | H | H | H | 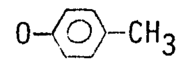 | H | a(Ex 3) | 42 | 121 |
| 4 | S | H | $CH_3$ | $CH_3$ | H | H | H | 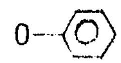 | $C_2H_5$ | b(Ex 2) | 39 | $^1H$ NMR |
| 5 | S | H | $OCH_3$ | H | H | H | H | 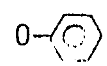 | $C_2H_5$ | b(Ex 2) | 35 | $^1H$ NMR |
| 6 | SO | H | $OCH_3$ | H | H | H | H | 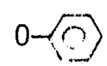 | $C_2H_5$ | a(Ex 1) | 44 | 141 |
| 7 | S | H | $OCH_3$ | H | H | H | H | 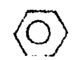 | H | b(Ex 2) | 61 | $^1H$ NMR |
| 8 | SO | H | $OCH_3$ | H | H | H | H | 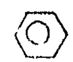 | H | a(Ex 1) | 79 | $^1H$ NMR |
| 9 | S | H | $OCH_3$ | H | H | H | H | 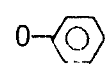 | H | b(Ex 2) | 87 | $^1H$ NMR |
| 10 | SO | H | $OCH_3$ | H | H | H | H | 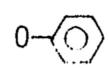 | H | a(Ex 1) | 57 | 118 |
| 11 | S | H | $CH_3$ | $CH_3$ | H | H | H | 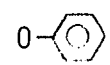 | H | b(Ex 2) | 69 | $^1H$ NMR |
| 12 | SO | H | $CH_3$ | $CH_3$ | H | H | H | 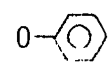 | H | a(Ex 1) | 53 | 115 |
| 13 | S | H | $CF_3$ | H | H | H | H | 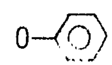 | H | b(Ex 2) | 81 | $^1H$ NMR |
| 14 | SO | H | $CF_3$ | H | H | H | H | 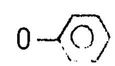 | H | a(Ex 1) | 38 | 151 |
| 15 | S | H | $OCH_3$ | H | H | H | $CH_3$ | 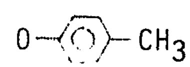 | H | b(Ex 2) | 63 | $^1H$ NMR |

cont.

| Ex | X | $R^1$ | $R^2$ | $R^3$ | $R^4$ | $R^5$ | $R^6$ | $R^7$ | $R^8$ | Method (Ex No) | Yield | M.p. (°C) other data |
|---|---|---|---|---|---|---|---|---|---|---|---|---|
| 16 | SO | H | $OCH_3$ | H | H | H | $CH_3$ | 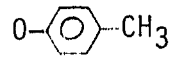 | H | a(Ex 1) | 38 | $^1H$ NMR |
| 17 | S | H | $OCH_3$ | H | H | H | H | 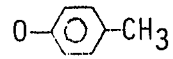 | $CH_3$ | b(Ex 2) | 79 | $^1H$ NMR |
| 18 | SO | H | $OCH_3$ | H | H | H | H | 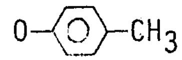 | $CH_3$ | a(Ex 1) | 77 | $^1H$ NMR |
| 19 | S | H | $OCH_3$ | H | H | H | H | 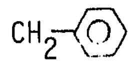 | H | b(Ex 2) | 96 | $^1H$ NMR |
| 20 | SO | H | $OCH_3$ | H | H | H | H | 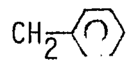 | H | a(Ex 3) | 55 | 74 |
| 21 | S | H | $OCH_3$ | H | H | H | H | 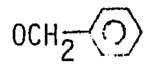 | $CH_3$ | b(Ex 2) | 56 | $^1H$ NMR |
| 22 | SO | H | $OCH_3$ | H | H | H | H | 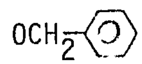 | $CH_3$ | a(Ex 3) | 55 | 111 |
| 23 | S | H | $OCH_3$ | H | H | H | H | 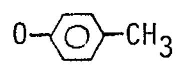 | H | b(Ex 2) | 77 | $^1H$ NMR |
| 24 | SO | H | $CH_3$ | $CH_3$ | H | H | H | 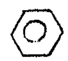 | H | a(Ex 1) | 73 | 170 |
| 25 | S | H | $OCH_3$ | H | H | H | H | 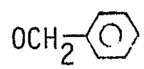 | $C_2H_5$ | b(Ex 2) | 63 | 162 |
| 26 | SO | H | $OCH_3$ | H | H | H | H | 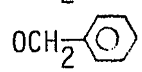 | $C_2H_5$ | a(Ex 1) | 39 | 77 |
| 27 | S | $CH_3$ | $C_2H_5$ | $CH_3$ | H | H | H | 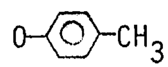 | $CH_3$ | | | |
| 28 | SO | $CH_3$ | $C_2H_5$ | $CH_3$ | H | H | H | 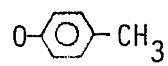 | $CH_3$ | | | |
| 29 | S | $CH_3$ | $COCH_3$ | $CH_3$ | H | H | H | 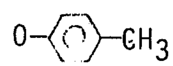 | $CH_3$ | | | |
| 30 | SO | $CH_3$ | $COCH_3$ | $CH_3$ | H | H | H | 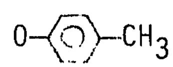 | $CH_3$ | | | |
| 31 | S | H | 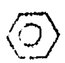 | H | H | H | $CH_3$ | 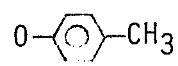 | H | | | |
| 32 | SO | H | 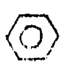 | H | H | H | $CH_3$ | 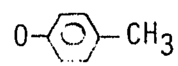 | H | | | |

cont.

cont.

| Ex | X | $R^1$ | $R^2$ | $R^3$ | $R^4$ | $R^5$ | $R^6$ | $R^7$ | $R^8$ | Method (Ex No) | Yield | M.p. (°C) other data |
|---|---|---|---|---|---|---|---|---|---|---|---|---|
| 33 | S | $CH_3$ | $OCH_2CH_2OCH_3$ | $CH_3$ | H | H | $CH_3$ | 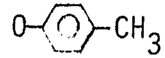 | H | | | |
| 34 | SO | $CH_3$ | $OCH_2CH_2OCH_3$ | $CH_3$ | H | H | $CH_3$ | 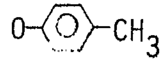 | H | | | |
| 35 | S | $CH_3$ | $CH_3$ | $CH_3$ | $CH_3$ | H | H | 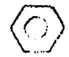 | H | | | |
| 36 | SO | $CH_3$ | $CH_3$ | $CH_3$ | $CH_3$ | H | H | 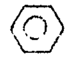 | H | | | |
| 37 | S | H | $C(CH_3)_3$ | H | H | H | H | 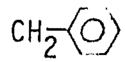 | H | b(Ex 2) | 69 | 67 |
| 38 | SO | H | $C(CH_3)_3$ | H | H | H | H | 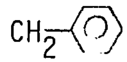 | H | a(Ex 1) | 20 | 119 |
| 39 | S | H | 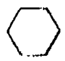 | H | H | H | H | 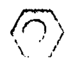 | H | | | |
| 40 | SO | H | 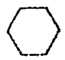 | H | H | H | H | 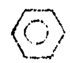 | H | | | |
| 41 | S | H | H | H | H | H | H | 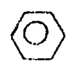 | H | | | |
| 42 | SO | H | H | H | H | H | H | 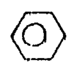 | H | | | |
| 43 | S | H | $OCH_3$ | H | H | H | $CH_3$ | 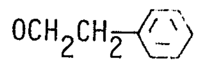 | $CH_3$ | b(Ex 2) | 72 | $^1H$ NMR |

Example 44. Preparation of 5-methoxy-2-[[(5-ethyl-4-phenoxy-2-pyridinyl)methyl]sulfinyl]-1*H*-benzimidazole sodium salt

NaOH (0.55 g, 0.0137 mol) is dissolved in 150 ml of methanol. To this solution 5-methoxy-2-[[(5-ethyl-4-phenoxy-2-pyridinyl)methyl]sulfinyl]-1*H*-benzimidazole (5.0 g, 0.0144 mol) is added and the mixture is stirred for 20 min at room temperature. After evaporation of the solvent the title compound is obtained in 99% yield.

Example 45. Preparation of 5-methoxy-2-[[(4-phenoxy-2-pyridinyl)methyl]sulfinyl]-1*H*-benzimidazole magnesium salt

5-Methoxy-2-[[(4-phenoxy-2-pyridinyl)methyl]sulfinyl]-1*H*-benzimidazole (2.9 g, 0.0077 mol) is dissolved in 50 ml of methanol. A solution of 0.34 g (0.0039 mol) of magnesium methoxide in 20 ml of methanol is added dropwise and then the mixture is stirred at room temperature for 20 min. The solvent is evaporated and the residue is suspended in ether, filtered off and dried giving the title compound in 86% yield.

Identifying data for compounds of the invention are given in the following Table 3.

Table 3. NMR-data of the compounds in Table 1

| Example No | NMR-data: $\delta(CDCl_3)$ ppm |
|---|---|
| 4 | 1.3(t, 3H), 2.48(s, 6H), 2.8(q, 2H), 4.2(s, 2H), 6.68(s, 1H), 7.05-7.65(m, 7H), 8.55(s, 1H). |
| 5 | 1.25(t, 3H), 2.75(q, 2H), 3.8(s, 3H), 4.15(s, 2H), 6.62(s, 1H), 6.75-7.55(m, 8H), 8.45(s, 1H). |
| 7 | 3.85(s, 3H), 4.45(s, 2H), 6.8-7.7(m, 10H), 8.7(d, 1H). |
| 8 | 3.75(s, 3H), 4.8(d, 2H), 6.85-7.65(m, 10H), 8.45(d, 1H). |
| 9 | 3.85(s, 3H), 4.32(s, 2H), 6.8-7.6(m, 10H), 8.55(d, 1H). |
| 11 | 2.3(s, 6H), 4.25(s, 2H), 6.75-7.5(m, 9H), 8.5(d, 1H). |
| 13 | 4.35(s, 2H), 6.8-7.9(m, 10H), 8.5(d, 1H). |
| 15 | 2.35(s, 3H), 2.4(s, 3H), 3.85(s, 3H), 4.45(2, 2H), 6.55(d, 1H), 6.8-7.55(m, 7H), 8.3(d, 1H). |
| 16 | 2.3(s, 3H), 2.4(s, 3H), 3.9(s, 3H), 4.85(s, 2H), 6.6(d, 1H), 6.9-7.4(m, 6H), 7.65(d, 1H), 8.3(d, 1H). |
| 17 | 2.3(s, 3H), 2.35(s, 3H), 3.9(s, 3H), 4.15(s, 2H), 6.63(s, 1H), 6.8-7.55(m, 7H), 8.45(s, 1H). |
| 18 | 2.2(s, 3H), 2.28(s, 3H), 3.85(s, 3H), 4.5(d, 2H), 6.32(s, 1H), 6.6-7.65(m, 7H), 8.32(s, 1H). |

NMR-data of the compounds in Table 1 cont.

| Example No | NMR-data: $\delta(CDCl_3)$ ppm |
|---|---|
| 43 | 2.1(s, 3H), 2.15(s, 3H), 3.1(t, 2H), 3.8(s, 3H), 4.02(t, 2H), 4.35(s, 2H), 6.8-7.55(m, 8H), 8.3(s, 1H). |
| 19 | 3.75(s, 3H), 3.82(s, 2H), 4.25(s, 2H), 6.8-7.35(m, 10H), 8.55(d, 1H). |
| 21 | 2.15(s, 3H), 3.75(s, 3H), 4.3(s, 2H), 5.02(s, 2H), 6.8-7.6(m, 9H), 8.32(s, 1H). |
| 23 | 2.32(s, 3H), 3.8(s, 3H), 4.32(s, 2H), 6.7-7.55(m, 9H), 8.48(d, 1H). |

Pharmaceutical preparations containing a compound of the invention as active ingredient are illustrated in the following formulations.

Syrup

A syrup containing 1% (weight per volume) of active substance was prepared from the following ingredients:

| | |
|---|---|
| Compound according to Example 45 | 1.0 g |
| Sugar, powder | 30.0 g |
| Saccharine | 0.6 g |
| Glycerol | 5.0 g |
| Flavouring agent | 0.05g |
| Ethanol 96% | 5.0 g |
| Distilled water q.s. to a final volume of | 100 ml |

Sugar and saccharine were dissolved in 60 g of warm water. After cooling the magnesium salt was dissolved in the sugar solution and glycerol and a solution of flavouring agents dissolved in ethanol were added. The mixture was diluted with water to a final volume of 100 ml.

Enteric-coated tablets

An enteric-coated tablet containing 20 mg of active compound was prepared from the following ingredients:

| | | |
|---|---|---|
| I | Compound according to Example 2, in neutral form | 200 g |
| | Lactose | 700 g |
| | Methyl cellulose | 6 g |
| | Polyvinylpyrrolidone cross-linked | 50 g |
| | Magnesium stearate | 15 g |
| | Sodium carbonate | 6 g |
| | Distilled water | q.s. |
| II | Cellulose acetate phthalate | 200 g |
| | Cetyl alcohol | 15 g |
| | Isopropanol | 2000 g |
| | Methylene chloride | 2000 g |

I The compound of Example 2, in neutral form, powder, was mixed with lactose and granulated with a water solution of methyl cellulose and sodium carbonate. The wet mass was forced through a sieve and the granulate dried in an oven. After drying the granulate was mixed with polyvinylpyrrolidone and magnesium stearate. The dry mixture was pressed into tablet cores (10 000 tablets), each tablet containing 20 mg of active substance, in a tabletting machine using 6 mm diameter punches.

II A solution of cellulose acetate phthalate and cetyl alcohol in isopropanol/methylene chloride was sprayed onto the tablets I in an Accela Cota®, Manesty coating equipment. A final tablet weight of 110 mg was obtained.

Solution for intravenous administration

A parenteral formulation for intravenous use, containing 4 mg of active compound per ml, was prepared from the following ingredients:

| | |
|---|---|
| Compound of Example 44 | 4 g |
| Polyethylene glycol 400 for injection | 400 g |
| Disodium hydrogen phosphate | q.s. |
| Sterile water to a final volume of | 1000 ml |

The sodium salt of the compound of Example 44 was dissolved in polyethylene glycol 400 and 550 ml of water was added. pH of the solution was brought to pH 7.4 by adding a water solution of disodium hydrogen phosphate and water was added to a final volume of 1000 ml. The solution was filtered through a 0.22 µm filter and immediately dispensed into 10 ml sterile ampoules. The ampoules were sealed.

## Biological tests

### Inhibiting effect *in vitro* on acid secretion in isolated rabbit gastric glands

### Test Method

### Gastric gland preparation

Isolated rabbit gastric glands were prepared as described by Berglindh et al., Acta physiol. scand. 1976. 96. 150-159. This method involves vascular perfusion of the rabbit stomach via the gastric arteries, scraping and scissor mincing of the separated gastric mucosa and collagenase (0.1%, Type I, Sigma Chemicals, St. Louis, MO. USA) digestion at 37°C for 60-90 min. The glands are then harvested and filtered through nylon cloth to remove coarse fragments. The glands are thereafter incubated at 37°C in a medium containing NaCl 132.4 mM, KCl 5.4 mM, $NaH_2PO_4$ 5.0 mM, $NaH_2PO_4$ 1.0 mM, $MgSO_4$ 1.2 mM, $CaCl_2$ 1.0 mM, glucose 10 mM, and 1 mg/ml rabbit albumine, pH 7.4.

### Measurement of acid secretion

The acid secretion in the isolated gland preparation was recorded by measuring the uptake of $^{14}C$-labelled aminopyrine into the glands as described by Berglindh et al., Acta physiol. scand. 1976. 97. 401-414. Accumulation of aminopyrine in the glands indicates gastric acid secretion within the glands. The standard medium contained $10^{-6}$M $^{14}C$-aminopyrine (Amersham, Great Britain). After the incubation period, the glands were centrifuged, the supernatant was removed and the glands dried, weighed and dissolved in Soluene -350 (Packard, IU. USA). Samples of the supernatant and glands were separately counted in a scintillation counter. The accumulation of $^{14}C$-labelled aminopyrine in the glands was calculated as detailed by Berglindh et al., Acta physiol. scand. 1976. 97. 403.

Experimental protocol

Glands were incubated for 60 min. in the presence of $5x10^{-5}$M histamine and the test compound to be studied. The free base of the test compound was dissolved in methanol. The final concentration of methanol was 1% in the incubation medium, having no influence on the aminopyrine accumulation ratio. For each test compound a complete dose-response curve was generated by testing doses in duplicate in the concentration range $10^{-7}$M to $10^{-4}$M. The negative logarithm of the concentration (in M) of the test compounds giving 50% inhibition of the aminopyrine accumulation in the glands ($pIC_{50}$) is listed in Table 4 below.

Table 4. Biological effects

| Tested compound<br>Example no. | Biological effect in glands<br>$pIC_{50}$ |
|---|---|
| 1 | 6.0 |
| 3 | 6.1 |
| 6 | 6.5 |
| 8 | 5.6 |
| 10 | 5.9 |
| 12 | 6.5 |
| 16 | 6.2 |
| 18 | 6.2 |
| 22 | 7.1 |
| 24 | 5.1 |
| 26 | 6.1 |

Comment to the test results

It is seen in Table 4 that the tested compounds potently inhibited gastric acid secretion.

Claims for the contracting states: BE CH DE FR GB IT LI LU NL SE:

1. A compound of the formula

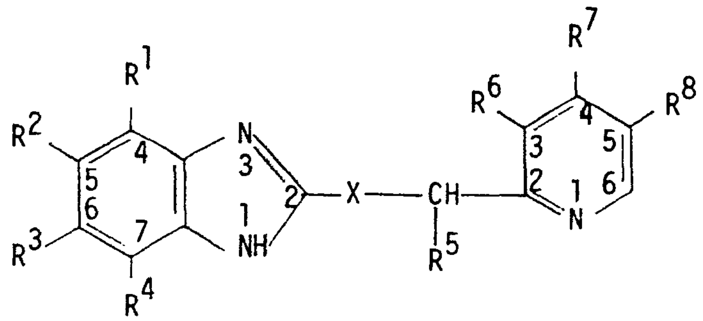

I

and therapeutically acceptable salts thereof, in which formula

X is -S- or $-\overset{O}{\overset{\uparrow}{S}}-$;

$R^1$, $R^2$, $R^3$ and $R^4$, which are the same or different, are

(a) H
(b) alkyl containing 1-6 carbon atoms
(c) cycloalkyl containing 3-7 carbon atoms
(d) alkoxy containing 1-6 carbon atoms
(e) alkoxyalkoxy containing 1-3 carbon atoms in each alkyl part
(f) halogen
(g) $CF_3$
(h) aryl
(i) aryloxy
or
(j) alkanoyl containing 2-7 carbon atoms;

$R^5$ is H, $CH_3$ or $C_2H_5$;

$R^6$ and $R^8$, which are the same or different, are
(a) H
(b) alkyl containing 1-6 carbon atoms;

and wherein
$R^7$ is 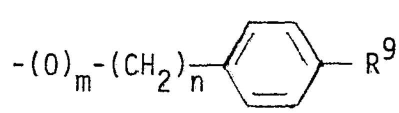

wherein

m is 0 or 1
n is 0, 1 or 2
$R^9$ is H or $CH_3$,

provided that when m, n, X, $R^1$, $R^2$, $R^3$, $R^4$, $R^5$, $R^6$ and $R^8$ are combined as follows: m is 1; n is 2; $R^9$ is H; X is SO; $R^1$, $R^3$ and $R^4$ are H; $R^6$ and $R^8$ are $CH_3$; and $R^5$ is H; then $R^2$ is other than $OCH_3$.

2. A compound according to claim 1, wherein the radicals $R^1$, $R^3$ and $R^4$ are H, and $R^2$ is other than H.

3. A compound according to claim 1 wherein $R^6$ and $R^8$ both are H.

4. A compound of the formula

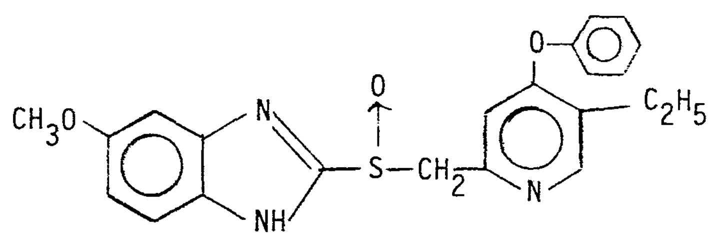

and its therapeutically acceptable salts.

5. A compound of the formula

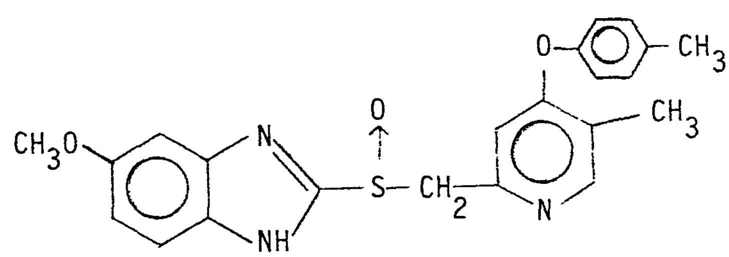

and its therapeutically acceptable salts.

6. A compound according to claim 1 wherein X is SO, in the form of an alkaline salt.

7. A pharmaceutical composition containing as active ingredient a compound according to any of claims 1-6.

8. A compound as defined in any of claims 1-6 or a therapeutically acceptable salt thereof for use as a medicament.

9. A compound as defined in any of claims 1-6 or a therapeutically acceptable salt thereof, for use in inhibiting gastric acid secretion in mammals and man.

10. A compound as defined in any of claims 1-6, or a therapeutically acceptable salt thereof, for use as gastrointestinal cytoprotecting agent in mammals and man.

11. A compound as defined in any of claims 1-6, or a therapeutically acceptable salt thereof, for use in the treatment of gastrointestinal inflammatory diseases in mammals and man.

12. A process for the preparation of a compound of the formula

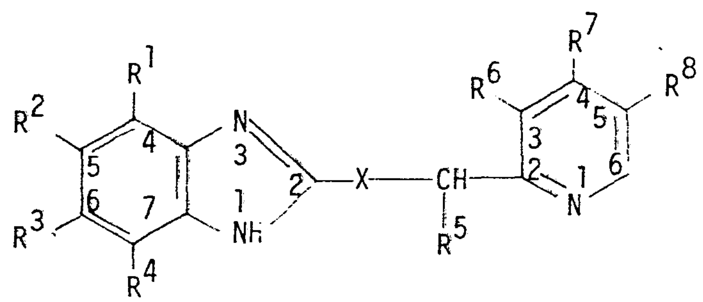

I

and therapeutically acceptable salts thereof, in which formula

X is -S- or $-\overset{O \atop \uparrow}{S}-$;

$R^1$, $R^2$, $R^3$ and $R^4$, which are the same or different, are

(a) H
(b) alkyl containing 1-6 carbon atoms
(c) cycloalkyl containing 3-6 carbon atoms
(d) alkoxy containing 1-6 carbon atoms
(e) alkoxyalkoxy containing 1-3 carbon atoms in each alkyl part
(f) halogen
(g) $CF_3$
(h) aryl
(i) aryloxy
or
(j) alkanoyl containing 2-7 carbon atoms;

$R^5$ is H, $CH_3$ or $C_2H_5$;

$R^6$ and $R^8$, which are the same or different, are
(a) H
(b) alkyl containing 1-6 carbon atoms;

and wherein
$R^7$ is $-(O)_m-(CH_2)_n-$ 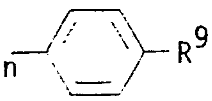

wherein

m is 0 or 1
n is 0, 1 or 2
$R^9$ is H or $CH_3$,

provided that when m, n, X, $R^1$, $R^2$, $R^3$, $R^4$, $R^5$, $R^6$ and $R^8$ are combined as follows: m is 1; n is 2; $R^9$ is H; X is SO; $R^1$, $R^3$, and $R^4$ are H; $R^6$ and $R^8$ are $CH_3$; and $R^5$ is H; then $R^2$ is other than $OCH_3$,

by

a) Oxidizing a compound of the formula I,

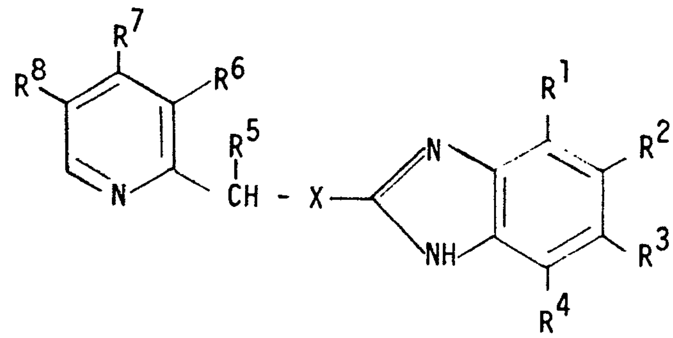

I

wherein X is S and $R^1$, $R^2$, $R^3$, $R^4$, $R^5$, $R^6$, $R^7$ and $R^8$ have the meanings given above, to give a compound of the same formula I wherein X is SO;

b) Reacting a compound of the formula

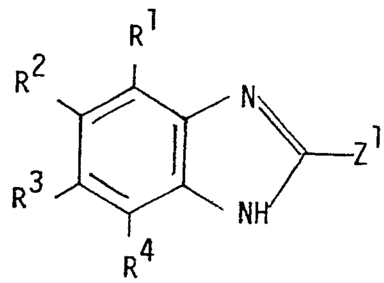

II

with a compound of the formula

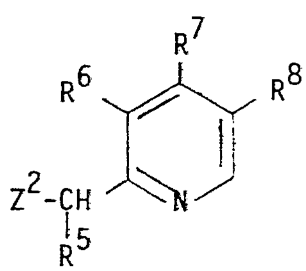

III

in which formulas $R^1$, $R^2$, $R^3$, $R^4$, $R^5$, $R^6$, $R^7$ and $R^8$ are as defined previously and wherein one of $Z^1$ and $Z^2$ is SH and the other is a leaving group, to give a compound of the formula I wherein X is S;

c) Hydrolyzing a compound of the formula

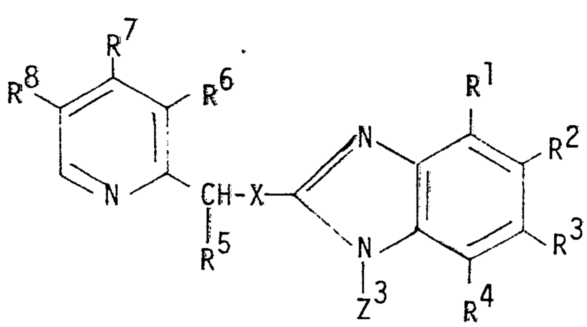

IV

wherein X, $R^1$, $R^2$, $R^3$, $R^4$, $R^5$, $R^6$, $R^7$ and $R^8$ are as defined above and $Z^3$ is a suitable N-protecting group such as alkanoyl, carboalkoxy and trimethylsilyl, to the formation of a compound of the formula I;

d) reacting a compound of the formula

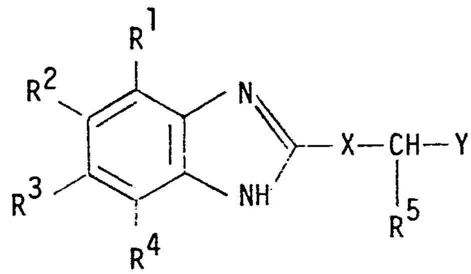

V

wherein $R^1$, $R^2$, $R^3$, $R^4$, $R^5$ and X are as defined above and Y is K, Na or Li,

with a compound of the formula

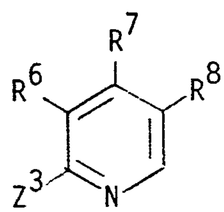

VI

wherein $R^6$, $R^7$ and $R^8$ are as defined above and $Z^3$ is halogen;

whereafter the compound of the formula I obtained, if desired, is converted to a physiologically acceptable salt.

13. A compound of the formula

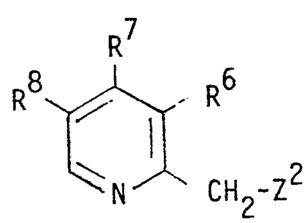

wherein $R^6$, $R^7$, and $R^8$ are as defined in claim 1 and $Z^2$ is SH or a leaving group.

14. A compound of the formula

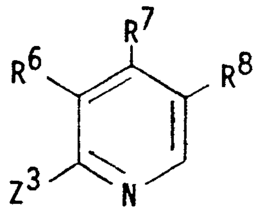

wherein $R^6$, $R^7$ and $R^8$ are as defined in claim 1 and $Z^3$ is halogen.

Claims for the contracting state AT:

1. A process for the preparation of a compound of the formula

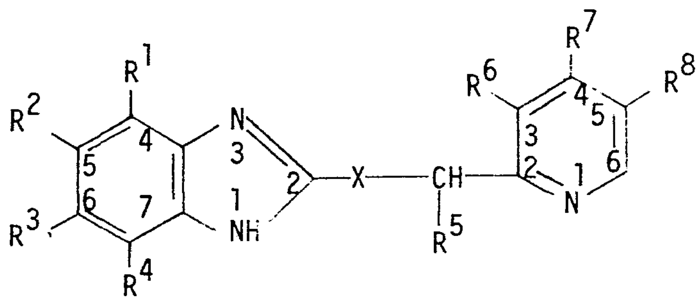

I

and therapeutically acceptable salts thereof, in which formula

X is -S- or $-\overset{O\uparrow}{S}-$;

$R^1$, $R^2$, $R^3$ and $R^4$, which are the same or different, are

(a) H
(b) alkyl containing 1-6 carbon atoms
(c) cycloalkyl containing 3-6 carbon atoms
(d) alkoxy containing 1-6 carbon atoms
(e) alkoxyalkoxy containing 1-3 carbon atoms in each alkyl part
(f) halogen
(g) $CF_3$
(h) aryl
(i) aryloxy
or
(j) alkanoyl containing 2-7 carbon atoms;

$R^5$ is H, $CH_3$ or $C_2H_5$;

$R^6$ and $R^8$, which are the same or different, are
(a) H
(b) alkyl containing 1-6 carbon atoms;

and wherein
$R^7$ is $-(O)_m-(CH_2)_n-$ 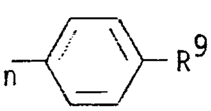

wherein

m is 0 or 1
n is 0, 1 or 2
$R^9$ is H or $CH_3$,

provided that when m, n, X, $R^1$, $R^2$, $R^3$, $R^4$, $R^5$, $R^6$ and $R^8$ are combined as follows: m is 1; n is 2; $R^9$ is H; X is SO; $R^1$, $R^3$, and $R^4$ are H; $R^6$ and $R^8$ are $CH_3$; and $R^5$ is H; then $R^2$ is other than $OCH_3$,

by

a) Oxidizing a compound of the formula I,

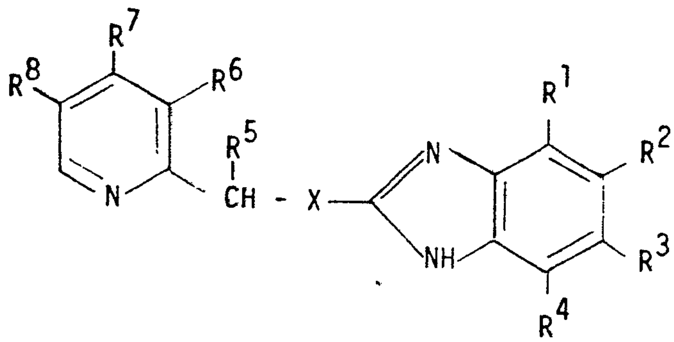

I

wherein X is S and $R^1$, $R^2$, $R^3$, $R^4$, $R^5$, $R^6$, $R^7$ and $R^8$ have the meanings given above, to give a compound of the same formula I wherein X is SO;

b) Reacting a compound of the formula

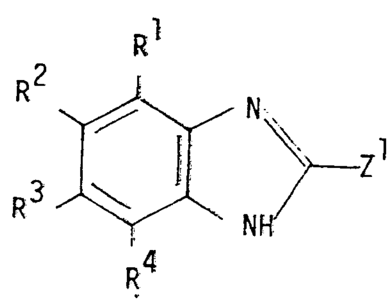

II

with a compound of the formula

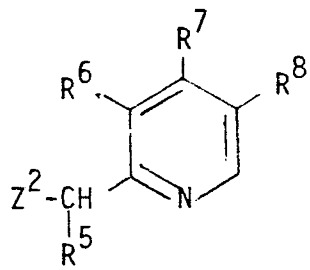

III

in which formulas $R^1$, $R^2$, $R^3$, $R^4$, $R^5$, $R^6$, $R^7$ and $R^8$ are as defined previously and wherein one of $Z^1$ and $Z^2$ is SH and the other is a leaving group, to give a compound of the formula I wherein X is S;

c) Hydrolyzing a compound of the formula

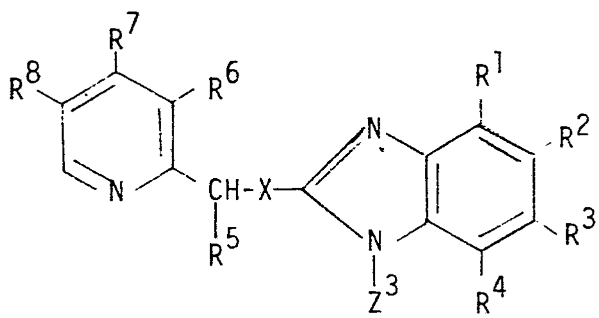

IV

wherein X, $R^1$, $R^2$, $R^3$, $R^4$, $R^5$, $R^6$, $R^7$ and $R^8$ are as defined above and $Z^3$ is a suitable N-protecting group such as alkanoyl, carboalkoxy and trimethylsilyl, to the formation of a compound of the formula I;

d) reacting a compound of the formula

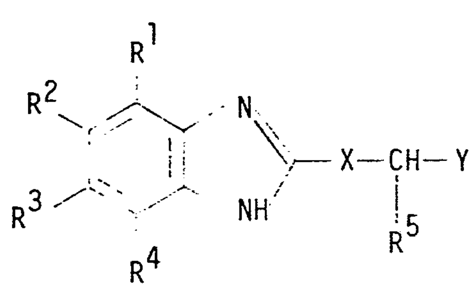

V

wherein $R^1$, $R^2$, $R^3$, $R^4$, $R^5$ and X are as defined above and Y is K, Na or Li,

with a compound of the formula

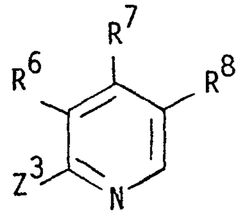

VI

wherein $R^6$, $R^7$ and $R^8$ are as defined above and $Z^3$ is halogen;

whereafter the compound of the formula I obtained, if desired, is converted to a physiologically acceptable salt.

2. A process according to claim 1 for the preparation of a compound according to formula I wherein the radicals $R^1$, $R^3$ and $R^4$ are H, and $R^2$ is other than H.

3. A process according to claim 1 for the preparation of a compound according to formula I wherein $R^6$ and $R^8$ both are H.

4. A process according to claim 1 for the preparation of a compound of the formula

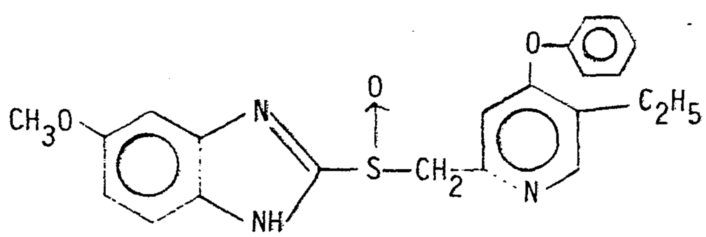

and its therapeutically acceptable salts.

5. A process according to claim 1 for the preparation of a compound of the formula

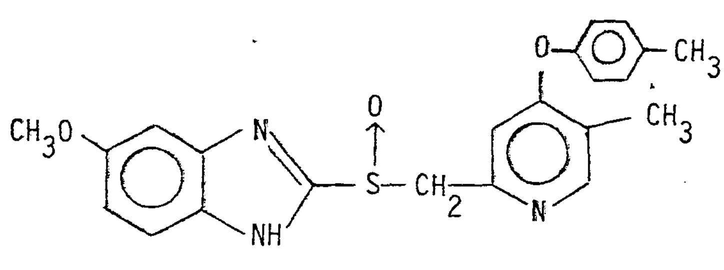

and its therapeutically acceptable salts.

6. A process according to claim 1 for the preparation of a compound according to formula I wherein X is SO, in the form of an alkaline salt.